# EUROPEAN PATENT SPECIFICATION

(11) **EP 3 854 402 B1**
(45) Date of publication and mention of the grant of the patent: **27.12.2023**
(21) Application number: 21150893.2
(22) Date of filing: 28.09.2016
(51) Int. Cl.: A61K 31/529, A61K 31/5355, A61K 31/56, A61K 39/395, A61K 45/06, A61P 37/06

(54) **TREATMENT OR PREVENTION OF GRAFT VERSUS HOST DISEASE**
BEHANDLUNG ODER PRÄVENTION VON TRANSPLANTAT-GEGEN-WIRT-ERKRANKUNG
TRAITEMENT OU PRÉVENTION DE LA MALADIE DU GREFFON CONTRE L'HÔTE

(30) Priority: 28.09.2015 US 201562233715 P
(43) Date of publication of application: 28.07.2021
(62) Divisional of application: 16852514.5
(73) Proprietor: CTI Biopharma Corp., Seattle, Washington 98121 (US)
(72) Inventor: Betts, Brian C., Seattle, WA Washington 98121 (US); Yu, Xuezhong, Seattle, WA Washington 98121 (US); Anasetti, Claudio, Seattle, WA Washington 98121 (US)
(74) Representative: Hoffmann Eitle

(56) References cited:
- WO-A1-2007/058627
- WO-A1-2012/022737
- CN-B- 102 617 599
- US-A1- 2014 357 557
- B. C. BETTS ET AL: "Janus kinase-2 inhibition induces durable tolerance to alloantigen by human dendritic cell-stimulated T cells yet preserves immunity to recall antigen", BLOOD, vol. 118, no. 19, 10 November 2011 (2011-11-10), pages 5330-5339, XP055386417, ISSN: 0006-4971, DOI: 10.1182/blood-2011-06-363408
- GUPTA VIKAS ET AL: "Janus Kinase Inhibitors and Allogeneic Stem Cell Transplantation for Myelofibrosis", BIOLOGY OF BLOOD AND MARROW TRANSPLANTATION, vol. 20, no. 9, 2014, pages 1274-1281, XP029040952, ISSN: 1083-8791, DOI: 10.1016/J.BBMT.2014.03.017
- HATZIMICHAEL ELEFTHERIA ET AL: "Profile of pacritinib and its potential in the treatment of hematologic disorders", JOURNAL OF BLOOD MEDICINE, 1 January 2014 (2014-01-01), pages 143-152, XP055804992, DOI: 10.2147/JBM.S51253 Retrieved from the Internet: URL:https://www.dovepress.com/getfile.php? fileID=21251>

## Description

The present invention relates to a compound or a composition comprising the compound for use in a method of treating or preventing graft-versus-host disease (GvHD) in a mammal, as defined in the appended claims.

### BACKGROUND OF THE DISCLOSURE

Solid organ transplant rejection occurs when transplanted tissue is rejected by the recipient's immune system, which destroys the transplanted tissue. Typical treatments for solid organ transplant rejection include a short course of high-dose corticosteroids, often in conjunction with an anti-proliferative agent. Antibody treatments have also been added to immunosuppressive therapies. However, certain antibodies, such as OKT3 are contraindicated because they trigger cytokine release syndrome and post-transplant lymphoproliferative disorders.

Graft-versus-host disease (GvHD) is a common complication following an allogeneic tissue transplant and is often associated with stem cell or bone marrow transplant. Essentially, GvHD occurs when mature transplanted lymphocytes entering the host with transplanted marrow recognize the new host tissues as foreign and destroy the host's tissues. GvHD affects about 50% of patients who survive beyond the first 100 days post-transplant. Acute GvHD generally targets the liver, skin, mucosa and the gastrointestinal tract as well as the hematopoietic system and the lungs, while chronic GvHD impacts the same organs as well as connective tissue and exocrine glands. Acute GvHD (hereinafter "aGvHD") is normally observed within the first 100 days post-transplant and chronic GvHD (hereinafter "cGvHD") normally occurs after 100 days. CN-B-102617599 discloses macrocyclic pyrimidines, which are inhibitors of JAK, such as of JAK2, for use in treating transplant rejection. Betts et al. describe in Blood, 118(19), 2011, 5330-5339 JAK2 inhibitors for use in the treatment of GvHD and solid organ allograft rejection.

### SUMMARY OF THE DISCLOSURE

The present invention is defined by the appended claims. The references to methods of treatment in this description are to be interpreted as references to the compounds, pharmaceutical compositions and medicaments of the present invention for use in a method for treatment of the human (or animal) body by therapy (or for diagnosis).

Disclosed herein (but not part of the claimed invention) are methods of treating solid organ transplant rejection and graft-versus-host disease (GvHD) in a patient in need thereof comprising administering a therapeutically effective amount of a Janus Kinase (JAK) inhibitor (e.g. a JAK2). In some non-claimed embodiments, the disclosure provides methods of treating solid organ transplant rejection and GvHD in a patient, comprising administering to a patient in need thereof a therapeutically effective amount of a compound of Formula (I) having the structure: 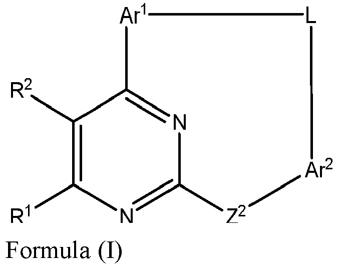 wherein:
R¹ and R² are H;
Z² is-N(H)-;
   Ar¹ is selected from the group consisting of: 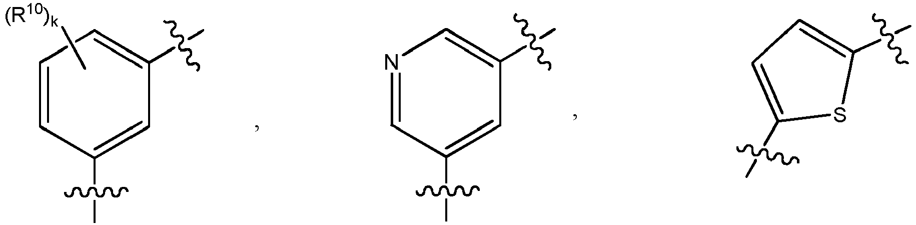 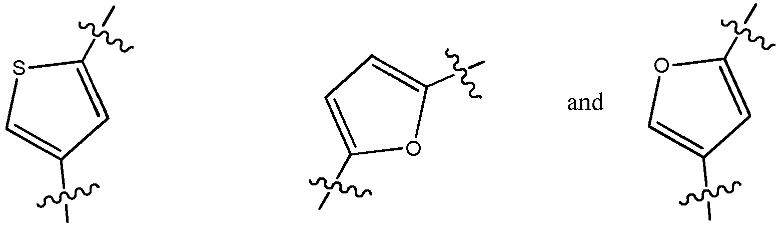
wherein R¹⁰ is methoxy or fluorine;
k is an integer selected from 0 or 1;
Ar² is a group of the formula 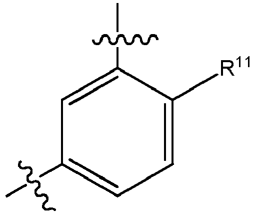
wherein R¹¹ is H or selected from the group consisting of: 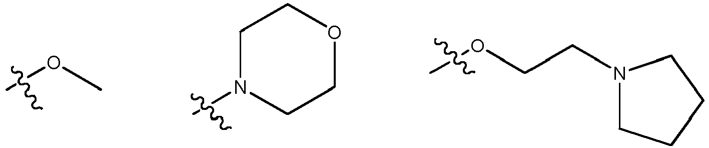 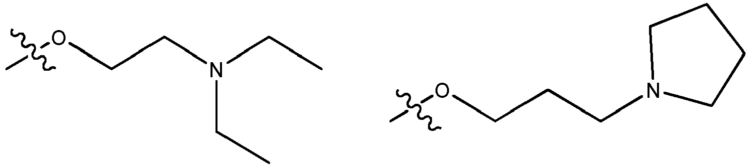 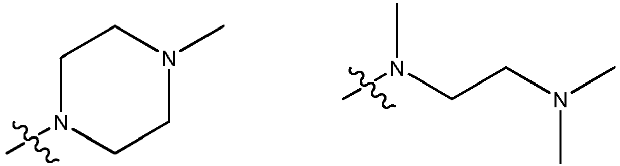 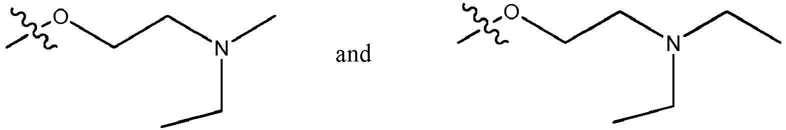
L is a group of formula:

   -X¹-Y-X²-
wherein X¹ is attached to Ar¹ and X² is attached to Ar², and wherein X¹, X² and Y are selected such that the group L has between 5 and 15 atoms in the normal chain,
wherein X¹ is selected from the group consisting of:
   (a)

      -OCH₂-
   (b)

      -OCH₂CH₂-,

      and
   (c)

      -CH₂OCH₂-;
wherein X² is selected from the group consisting of:
   (a)

      -CH₂O-,
   (b)

      -CH₂CH₂O-,

      and
   (c)

      -CH₂OCH₂-;
Y is a group of formula -CR^{a}=CR^{b}-,
wherein R^{a} and R^{b} are H,
or a pharmaceutically acceptable salt or N-oxide thereof.

According to the invention, the compound of Formula (I) is 11-(2-Pyrrolidin1-yl-ethoxy)-14,19-dioxa-5,7,26-triazatetracyclo[19.3.1.1^{2,6}.1^{8,12}] heptacosa-1(25),2(26),3,5,8,10,12(27),16,21,23-decaene (pacritinib) or a pharmaceutically acceptable salt or N-oxide thereof, such as its citrate or maleate salts. In some embodiments, the patient exhibits one or more symptoms of solid organ transplant rejection or GvHD. In some embodiments, the GvHD is multi-organ GvHD. In some embodiments the patient has received a solid organ transplant. In some embodiments the solid organ transplant is lung, liver, kidney, heart, pancreas, skin, stomach, or vascularized composite grafts. In some embodiments the patient has received a cell transplant. In some embodiments, the cell transplantation is allogeneic bone marrow or hematopoetic stem cell transplant. In some embodiments, the compound of Formula (I) is administered concurrently with a solid organ transplant, an allogeneic bone marrow or hematopoietic stem cell transplant. In some embodiments, the compound of Formula (I) is administered subsequent to a solid organ transplant, an allogeneic bone marrow or a hematopoietic stem cell transplant. In some embodiments, the amount of the JAK inhibitor compound according to claim 1 prevents or reduces solid organ transplant rejection or GvHD while promoting inducible regulatory T-cell (iTreg) responses after allogeneic hematopoietic cell transplantation (allo-HCT). In some embodiments, the compound according to claim 1 is administered at a dosage of between about 0.1 mg/kg per day to about 1000 mg/kg per day. In some embodiments, the amount of the compound according to claim 1 administered is about 10 mg/day, about 100 mg/day, about 200 mg/day, about 400 mg/day, about 500 mg/day, about 600 mg/day or about 700 mg/day. In some embodiments, the compound according to claim 1 is administered once daily from day 1 to about day 2000 following solid organ transplant or allogeneic bone marrow or hematopoietic stem cell transplant. In some embodiments, the compound according to claim 1 is administered from the onset of transplant rejection symptoms to about day 2000, or until rejection symptoms resolve following solid organ transplant or allogeneic bone marrow or hematopoietic stem cell transplant. In other embodiments the compound according to claim 1 is administered twice per day, three times per day or four times per day. In some embodiments, the compound according to claim 1 is administered orally. In some embodiments, the compound according to claim 1 is administered in combination or conjunction with one or more additional therapeutic agents.

The present disclosure also provides compositions for preventing, treating or reducing the occurrence or reducing the severity of solid organ transplant rejection and GvHD in a patient in need thereof, comprising administering an effective amount of a compound of Formula (I) and a pharmaceutically acceptable carrier (not part of the claimed invention).

These and other embodiments of the disclosure will be readily apparent to those of ordinary skill in view of the disclosure herein.

### BRIEF DESCRIPTION OF THE DRAWINGS

Fig. 1 shows survival of GvHD mice. Plot provides overall survival for bone marrow (BM) transplantation of JAK2 -/- T cells is associated with significantly less GvHD compared with wild-type (WT) or JAK2 replete (JAK2 +/+) donors.
Fig. 2 shows survival of GvHD mice. Plot provides graft survival following peripheral blood mononuclear cells (PMBC) infusion-vehicle control vs. pacritinib administration 100mg/kg while representative images show skin at time of suture removal (day -30) and at day +35.
Fig. 3 shows H&E (top images) and CD3 (brown/bottom images) IHC staining of the skin grants at day +21, before the onset of graft rejection.

### DETAILED DESCRIPTION OF THE DISCLOSURE

Disclosed herein (but not part of the claimed invention) are methods of treating solid organ transplant rejection and graft-versus-host disease (GvHD) in a patient in need thereof comprising administering a therapeutically effective amount of a Janus kinase (JAK) inhibitor (e.g. a JAK2). In some non-claimed embodiments, the disclosure provides methods of treating solid organ transplant rejection and GvHD in a patient, comprising administering to a patient in need thereof a therapeutically effective amount of a compound of Formula (I) having the structure: 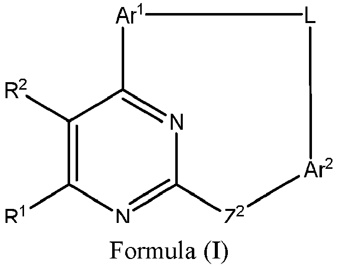 wherein:
R¹ and R² are H;
Z² is-N(H)-;
Ar¹ is selected from the group consisting of: 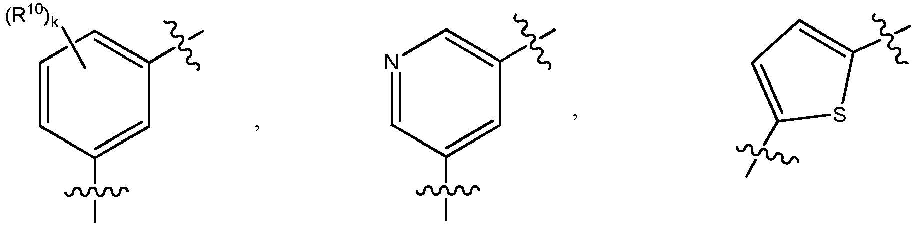 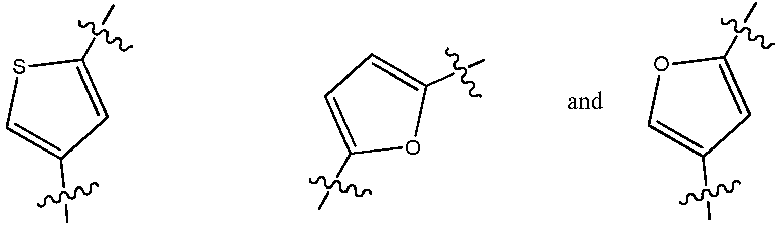
wherein R¹⁰ is methoxy or fluorine;
k is an integer selected from 0 or 1;
Ar² is a group of the formula 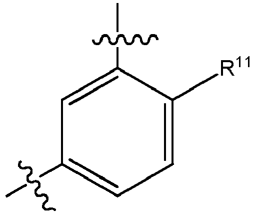
wherein R¹¹ is H or selected from the group consisting of: 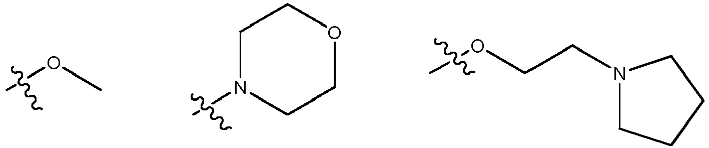 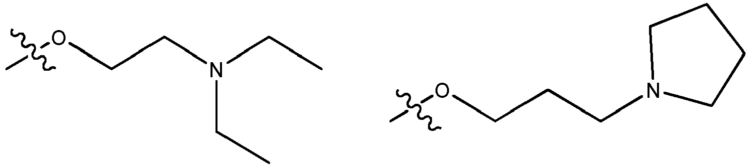 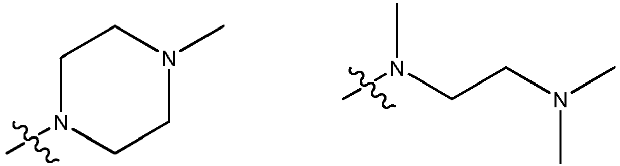 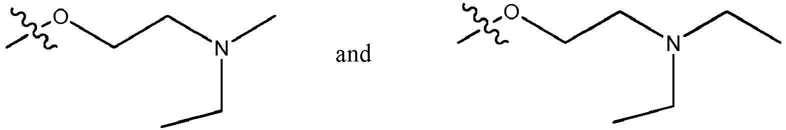
L is a group of formula:

   -X¹-Y-X²-
wherein X¹ is attached to Ar¹ and X² is attached to Ar², and wherein X¹, X² and Y are selected such that the group L has between 5 and 15 atoms in the normal chain,
wherein X¹ is selected from the group consisting of:
   (a)

      -OCH₂-
   (b)

      -OCH₂CH₂-,

      and
   (c)

      -CH₂OCH₂-;
wherein X² is selected from the group consisting of:
   (a)

      -CH₂O-,
   (b)

      -CH₂CH₂O-,

      and
   (c)

      -CH₂OCH₂-;
Y is a group of formula -CR^{a}=CR^{b}-,
wherein R^{a} and R^{b} are H,
or a pharmaceutically acceptable salt or N-oxide thereof. According to the invention, the compound of Formula (I) is 11-(2-Pyrrolidin1-yl-ethoxy)-14,19-dioxa-5,7,26-triazatetracyclo[19.3.1.1^{2,6}.1^{8,12}] heptacosa-1(25),2(26),3,5,8,10,12(27),16,21,23-decaene (pacritinib) or a pharmaceutically acceptable salt or N-oxide thereof, such as its citrate or maleate salts. In some embodiments, the patient exhibits one or more symptoms of solid organ transplant rejection or GvHD. In some embodiments, the GvHD is multi-organ GvHD. In some embodiments the patient has received a solid organ transplant. In some embodiments the solid organ transplant is lung, liver, kidney, heart, pancreas, skin, stomach, or vascularized composite grafts. In some embodiments the patient has received a cell transplant. In some embodiments, the cell transplantation is allogeneic bone marrow or hematopoetic stem cell transplant. In some embodiments, the compound according to claim 1 is administered concurrently with a solid organ transplant, an allogeneic bone marrow or a hematopoietic stem cell transplant. In some embodiments, the compound according to claim 1 is administered subsequent to a solid organ transplant, an allogeneic bone marrow or a hematopoietic stem cell transplant. In some embodiments, the amount of the JAK inhibitor compound according to claim 1 prevents or reduces solid organ transplant rejection or GvHD while promoting inducible regulatory T-cell (iTreg) responses after allogeneic hematopoietic cell transplantation (allo-HCT). In some embodiments, the compound according to claim 1 is administered at a dosage of between about 0.1 mg/kg per day to about 1000 mg/kg per day. In some embodiments, the amount of the compound according to claim 1 administered is about 10 mg/day, about 100 mg/day, about 200 mg/day, about 400 mg/day, about 500 mg/day, about 600 mg/day or about 700 mg/day. In some embodiments, the compound according to claim 1 is administered once daily from day 1 to about day 2000 following solid organ transplant or allogeneic bone marrow or hematopoietic stem cell transplant. In some embodiments, the compound according to claim 1 is administered from the onset of GvHD symptoms to about day 2000 following solid organ transplant or allogeneic bone marrow or hematopoietic stem cell transplant. In other embodiments the compound according to claim 1 is administered twice per day, three times per day or four times per day. In some embodiments, the compound according to claim 1 is administered orally. In some embodiments the compound according to claim 1 is administered parenterally. In some embodiments, the compound according to claim 1 is administered in combination or conjunction with one or more additional therapeutic agents.

The present disclosure also provides compositions for preventing, treating or reducing the occurrence or reducing the severity of solid organ transplant rejection and GvHD in a patient in need thereof, comprising administering an effective amount of a compound of Formula (I) and a pharmaceutically acceptable carrier (not part of the claimed invention).

The disclosure also provides a method of delaying allograft rejection in a transplant patient comprising administering to a patient in need thereof a therapeutically effective amount of a compound of Formula (I) as described above (not part of the claimed invention).

Disclosed (but not claimed) herein, in further embodiments, is a method of reducing T-cell proliferation in a transplant patient comprising administering to a patient in need thereof a therapeutically effective amount of a compound of Formula (I) as described above.

In this specification a number of terms are used which are well known to a skilled person. Nevertheless for the purposes of clarity a number of terms are defined hereinbelow.

It is understood that included in the family of compounds of Formula (I) used in the methods of the present disclosure are isomeric forms including diastereoisomers, enantiomers, tautomers, and geometrical isomers in "E" or "Z" configurational isomer or a mixture of E and Z isomers. It is also understood that some isomeric forms such as diastereomers, enantiomers, and geometrical isomers can be separated by physical and/or chemical methods and by those skilled in the art.

Some of the compounds of the disclosed embodiments used in practicing the methods of the present invention may exist as single stereoisomers, racemates, and/or mixtures of enantiomers and/or diastereomers. All such single stereoisomers, racemates and mixtures thereof, are intended to be within the scope of the subject matter described and claimed. Additionally, Formula (I) is intended to cover, where applicable, solvated as well as unsolvated forms of the compounds.

Thus, each formula includes compounds having the indicated structure, including the hydrated as well as the non-hydrated forms.

In addition to the compounds of Formula (I), the compounds of the various embodiments used in practicing the methods of the present disclosure include pharmaceutically acceptable salts, prodrugs, N-oxides and active metabolites of such compounds, and pharmaceutically acceptable salts of such metabolites.

The term "pharmaceutically acceptable salts" refers to salts that retain the desired biological activity of the above- identified compounds, and include pharmaceutically accept able acid addition salts and base addition salts. Suitable pharmaceutically acceptable acid addition salts of compounds of Formula (I) used in practicing the methods of the present invention may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of Formula (I) include metallic salts made from lithium, sodium, potassium, magnesium, calcium, aluminium, and zinc, and organic salts made from organic bases such as choline, diethanolamine, morpholine. Other examples of organic salts are: ammonium salts, quaternary salts such as tetramethylammonium salt; amino acid addition salts such as salts with glycine and arginine. Additional information on pharmaceutically acceptable salts can be found in Remington's Pharmaceutical Sciences, 19th Edition, Mack Publishing Co., Easton, Pa. 1995. In the case of agents that are solids, it is understood by those skilled in the art that the inventive compounds, agents and salts may exist in different crystalline or polymorphic forms, all of which are intended to be within the scope of the present invention and specified formulae.

"Prodrug" (not part of the claimed invention) means a compound which is convertible in vivo by metabolic means (e.g. by hydrolysis, reduction or oxidation) to a compound of formula (I). For example an ester prodrug of a compound of formula (I) containing a hydroxyl group may be convertible by hydrolysis in vivo to the parent molecule. Suitable esters of compounds of formula (I) containing a hydroxyl group are for example acetates, citrates, lactates, tartrates, malonates, oxalates, salicylates, propionates, succinates, fumarates, maleates, methylene-bis-p-hy- droxynaphthoates, gestisates, isothionates, di-p-toluoyltartrates, methanesulphonates, ethanesulphonates, benzenesulphonates, p-toluenesulphonates, cyclohexylsulphamates and quinates. As another example an ester prodrug of a compound of formula (I) containing a carboxy group may be convertible by hydrolysis in vivo to the parent molecule.

The term "therapeutically effective amount" or "effective amount" is an amount sufficient to effect beneficial or desired clinical results. An effective amount can be administered in one or more administrations. An effective amount is typically sufficient to palliate, ameliorate, stabilize, reduce, reverse, treat, slow or delay the progression of the disease state (e.g. solid organ transplant rejection or GvHD).

Specific compounds used in practicing the methods of the present invention include the following: 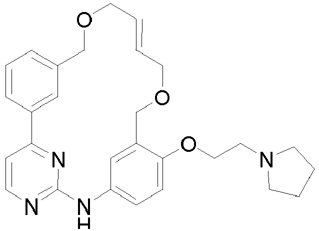 or a pharmaceutically acceptable salt thereof.

The compound of Formula (I) is 11-(2-Pyrrolidin1-yl-ethoxy)-14,19-dioxa-5,7,26-triazatetracyclo[19.3.1.1^{2,6}.1^{8,12}] heptacosa-1(25),2(26),3,5,8,10,12(27),16,21,23-decaene (pacritinib) or a pharmaceutically acceptable salt or N-oxide thereof, such as its citrate or maleate salts.

Administration of compounds within Formula (I) to humans for purposes of preventing, treating or reducing the occurrence or reducing the severity of solid organ transplant rejection and GvHD can be by any of the accepted modes for enteral administration such as oral or rectal, or by parenteral administration such as subcutaneous, intramuscular, intravenous and intradermal routes. Injection can be bolus or via constant or intermittent infusion. The active compound is typically included in a pharmaceutically acceptable carrier or diluent and in an amount sufficient to deliver to the patient a therapeutically effective dose.

The compounds used in practicing the methods of the present invention can be administered in any form or mode which makes the compound bioavailable. One skilled in the art of preparing formulations can readily select the proper form and mode of administration depending upon the particular characteristics of the compound selected, the condition to be treated and other relevant circumstances. The reader is referred to Remingtons Pharmaceutical Sciences, 19th edition, Mack Publishing Co. (1995) for further information. The compounds used in practicing the methods of the present invention can be administered alone or in the form of a pharmaceutical composition in combination with a pharmaceutically acceptable carrier, diluent or excipient. The compounds used in practicing the methods of the present invention, while effective themselves, are typically formulated and administered in the form of their pharmaceutically acceptable salts as these forms are typically more stable, more easily crystallized and have increased solubility. The compounds used in practicing the methods of the present invention are also typically used in the form of pharmaceutical compositions which are formulated depending on the desired mode of administration. The compositions are prepared in manners well known in the art.

The compounds used in practicing the methods of the present invention may be used or administered in combination with one or more additional drug(s), e.g. anti-rejection drugs that are suitable for the treatment of or reduction of the symptoms of solid organ transplant rejection and/or GvHD. Representative drugs which may be used or administered in combination with the compounds used in practicing the methods of the present invention include: corticosteroids, such as prednisone or methylprednisone; calcineurin inhibitors, such as cyclosporine and tacrolimus; mycophenolate mofetil; antiproliferative agents such as azathiprine, everolimus and sirolimus; monoclonal antibodies such as basiliximab, dacilzumab, and rituximab; and polyclonal antibodies such as antithymocite globulin-equine and antithymocyte globulin-rabbit. The components can be administered in the same formulation or in separate formulations. If administered in separate formulations the compounds used in practicing the methods of the present invention may be administered sequentially or simultaneously with the other drug(s). In addition to being able to be administered in combination with one or more additional drugs that include anti-rejection drugs, the compounds used in practicing the methods of the present invention may be used in a combination therapy. When this is done the compounds are typically administered in combination with each other. Thus, one or more of the compounds used in practicing the methods of the present invention may be administered either simultaneously (as a combined preparation) or sequentially in order to achieve a desired effect. This is especially desirable where the therapeutic profile of each compound is different such that the combined effect of the two drugs provides an improved therapeutic result.

Pharmaceutical compositions for parenteral injection and used in practicing the methods of the present invention comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions used in practicing the methods of the present invention may also contain adjuvants such as preservative, wetting agents, emulsifying agents, and dispersing agents. Prevention of the action of micro-organisms may be ensured by the inclusion of various antibacterial and anti-fungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminium monostearate and gelatin. If desired, and for more effective distribution, the compounds can be incorporated into slow release or targeted delivery systems such as polymer matrices, liposomes, and microspheres.

The injectable formulations can be sterilized, for example, by filtration through a bacteria-retaining filter, or by incorporating sterilizing agents in the form of sterile solid compositions that can be dissolved or dispersed in sterile water or other sterile injectable medium just prior to use.

Solid dosage forms for oral administration and used in practicing the methods of the present invention include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol and silicic acid, b) binders such as, for example, carboxymethylcellulose, alginates, gelatin, polyvinylpyrrolidone (PVP), sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, capsules, pills, and granules can be prepared with coatings and shells such as enteric coatings and other coatings well known in the phar- maceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

If desired, and for more effective distribution, the compounds used in practicing the methods of the present invention can be incorporated into slow release or targeted delivery systems such as polymer matrices, liposomes, and microspheres.

The active compounds used in practicing the methods of the present invention can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, ground- nut, com, germ, olive, castor, and sesame oils), glycerol, tetrahydroforfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents. Dosage forms for topical administration of a compound of this invention include powders, patches, sprays, ointments and inhalants. The active compound used in practicing the methods of the present invention is mixed under sterile conditions with a pharmaceutically acceptable carrier and any needed preservatives, buffers, or propellants which may be required.

The amount of compound administered in practicing the methods of the present invention will preferably treat and reduce or alleviate the condition. A therapeutically effective amount can be readily determined by an attending diagnostician or physician or other health care worker by the use of conventional techniques and by observing results obtained under analogous circumstances. In determining the therapeutically effective amount a number of factors are to be considered including but not limited to, the species of animal, its size, age and general health, the specific condition involved, the severity of the condition, the response of the patient to treatment, the particular compound administered, the mode of administration, the bioavailability of the preparation administered, the dose regime selected, the use of other medications and other relevant circumstances.

A preferred dosage will be a range from about 0.01 to 1000 mg per kilogram of body weight per day. A more preferred dosage will be in the range from 0.1 to 100 mg per kilogram of body weight per day, more preferably from 0.2 to 80 mg per kilogram of body weight per day, even more preferably 0.2 to 50 mg per kilogram of body weight per day. A suitable dose can be administered in multiple sub-doses per day.

Described herein (but not part of the claimed invention), in some embodiments, are methods of preventing, treating or reducing the occurrence or severity of solid organ transplant rejection and graft versus host disease (GvHD) in a patient in need thereof, comprising administering a therapeutically effective amount of a JAK inhibitor (e.g., a JAK2 inhibitor). Further described herein (but not part of the claimed invention), are methods of reducing the occurrence of graft versus host disease or reducing the severity of GvHD occurrence in a patient requiring cell transplantation comprising administering to the patient a composition comprising a therapeutically-effective amount of an JAK inhibitor compound (e.g., a JAK2 inhibitor, such as, for example, pacritinib). In one embodiment the GvHD is alloantibody driven GvHD which develops in part due to alloantibody production following an allogeneic transplant, such as a hematopoietic stem cell transplant. In some embodiments, the patient has received a peripheral blood stem cell transplantion. In some embodiments, the patient has received a bone marrow transplantation. In some embodiments the JAK inhibitor compound (e.g. pacritinib) is administered prior to administration of the cell transplant. In another embodiment, the JAK inhibitor compound as administered subsequent to administration of the cell transplant. In yet another embodiment, the JAK inhibitor compound is administered concurrently with administration of the cell transplant.

Described herein are methods of preventing, treating or reducing the occurrence of alloantibody driven GvHD or reducing the severity of alloantibody driven GvHD occurrence in a patient requiring stem cell transplantation comprising administering to the patient a composition comprising a therapeutically-effective amount of pacritinib.

Described herein (but not part of the claimed invention), in some embodiments, are methods of treating alloantibody driven graft versus host disease (GvHD) and solid organ transplant rejection in a patient in need thereof, comprising administering a therapeutically effective amount of a JAK inhibitor (e.g., a JAK2 inhibitor). Further described herein (but not part of the claimed invention), are methods of preventing the occurrence of graft versus host disease (GvHD) or reducing the severity of GvHD occurrence in a patient requiring cell transplantation comprising administering to the patient a composition comprising a therapeutically-effective amount of a JAK inhibitor compound (e.g., a JAK2 inhibitor, such as, for example, pacritinib).

Further described herein (but not part of the claimed invention), are methods of treating a patient for alleviation of a bone marrow mediated disease, with alleviation of consequently developed graft versus host disease (GvHD), comprising administering to the patient allogeneic hematopoietic stem cells and/or allogeneic T-cells, wherein a therapeutically effective amount of an JAK inhibitor compound (e.g., a JAK2 inhibitor, such as, for example, pacritinib) is administered prior to or concurrently with the allogeneic hematopoietic stem cells and/or allogeneic T-cells.

### EXAMPLE

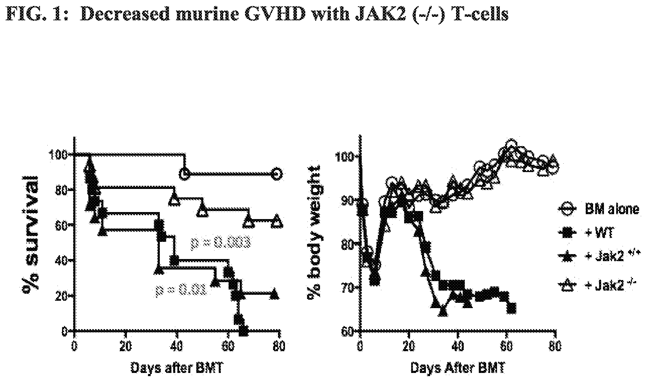

To determine whether pacritinib could treat established GvHD, a murine model of allogeneic hematopoetic cell transplantation was utilized. In addition, in order to determine whether JAK 2 inhibition could prevent or reduce the severity of solid organ graft rejection, a human skin/NSG mouse xenograft model was employed. Using murine models of allo-HCT the inventors demonstrated that transfer of donor JAK2^{-/-} T cells is associated with significantly less GVHD, compared with wild-type or JAK2 replete donors (See Figure 1, P=.003 and 0.01, respectively). Th1 differentiation among JAK2^{-/-} T-cells is dramatically decreased, compared with controls. Conversely, iTreg polarization and stability are significantly increased among the JAK2 deficient T-cells.

To investigate the effect of pharmacologic JAK2 inhibition on T-cell alloresponses, pacritinib (supplied by CTI BioPharma), was chosen as it does not induce myelosuppression or increase risk for opportunistic infections in myelofibrosis patients - unlike JAK1/JAK2 inhibitors. Pacritinib potently inhibits JAK2, but also has suppressive activity toward JAK3, CSF1R, and IRAK1. Pacritinib was administered at 100mg/kg twice a day by oral gavage for 4 weeks beginning on the day of MHC-disparate allo-HCT, significantly reducing GVHD in recipient mice.

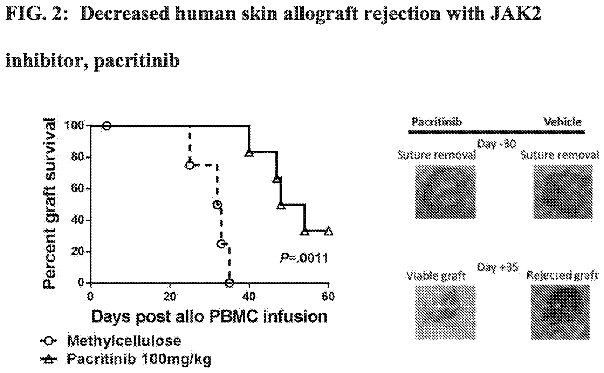

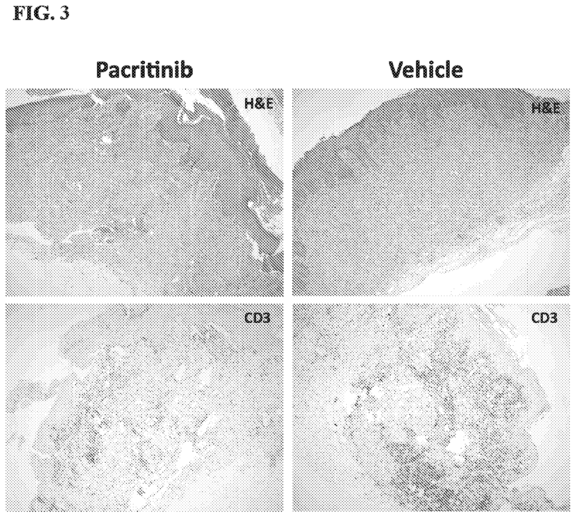

The inventors then evaluated whether JAK2 inhibition could prevent solid organ graft rejection, using a human skin / NSG mouse xenograft model. A 1x1 cm split-thickness human skin graft was transplanted onto the animal dorsally, followed by intraperitoneal injection of 5×10⁶ allogeneic peripheral blood mononuclear cells (PBMC) 30 days after surgery. Pacritinib was administered 100mg/kg twice a day by oral gavage for 15 days beginning at time of PBMC injection as tolerated by the NSG mice. The treatment significantly delayed allograft and xenograft rejection by the human donor PBMCs, compared with vehicle control (See Figure 2: Median graft survival 32.5 v 51 days, Day +60 survival 0% v 33.3%, pooled data from 2 experiments, n=5-6 mice per arm, P=.0011; See Figure 3: representative sections of skin grafts at day +21 show that pacritinib reduces lymphocytic infiltration of the tissue (H&E (top images) and CD3 [brown/bottom images]).

In allogeneic mixed leukocyte reactions using human cells, pacritinib (2.5µM) significantly reduced T-cell proliferation after 5 days of culture (Mean proliferation 100% v 23.7%, DC:T-cell ratio 1:30, n=4 in triplicate, P< 0001). *In vitro* studies verified pacritinib (2.5µM) eliminated STAT3 activity by IL-6 in human CD4+ T-cells, while permitting IL-2 induced STATS phosphorylation despite its effects on JAK3. This offers a platform to reduce Th17 development, while promoting Treg populations. As such, pacritinib dramatically suppresses Th17 differentiation among naive CD4+ T-cells stimulated by allogeneic dendritic cells (DC), compared with DMSO (relative RORgammaT expression 1 v 0.12, n=2 in triplicate, P=<.0001). iTregs were generated with allogeneic DCs in the presence of pacritinib or DMSO for 5 days, and then cultured with self T-cell responders and fresh DCs without additional drug exposure. The suppressive potency of either pacritinib- or DMSO-treated iTregs was similar, suggesting JAK2 is not required for iTreg function. Collectively, these data clearly identify JAK2 as a therapeutic target to control donor alloreactivity and promote iTreg responses after allogeneic hematopoietic cell transplantation (HCT) or solid organ transplantation.

## Claims

1. A compound or a composition comprising the compound for use in a method of treating or preventing GvHD in a mammal, the compound having the following structure: 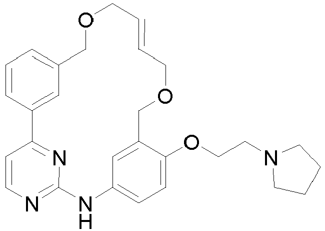 or a pharmaceutically acceptable salt or N-oxide thereof.

2. The compound or composition for use according to claim 1, **characterized in that** the compound or composition is for administration concurrently with an allogeneic bone marrow or hematopoietic stem cell transplant.

3. The compound or composition for use according to claim 1, **characterized in that** the compound or composition is for administration sequentially with an allogeneic bone marrow or hematopoietic stem cell transplant.

4. The compound or composition for use according to claim 1, **characterized in that** the compound or composition is for administration subsequent to an allogeneic bone marrow or hematopoietic stem cell transplant.

5. The compound or composition for use according to claim 1, wherein the compound or composition is for administration once daily from day 1 to about day 2000 following allogeneic bone marrow or hematopoietic stem cell transplant.

6. The compound or composition for use according to claim 1, wherein the compound or composition is for administration from the onset of GvHD symptoms to about day 2000 following allogeneic bone marrow or hematopoietic stem cell transplant.

7. The compound or composition for use according to claim 1, wherein the compound or composition is administered at a dosage of between about 0.1 mg/kg per day to about 1000 mg/kg per day, preferably about 100 mg/kg per day.

8. The compound or composition for use according to claim 1, wherein the compound or composition is formulated for oral administration.

9. The compound or composition for use according to claim 1, wherein the compound or composition is formulated for administration twice per day.

10. The compound or composition for use according to claim 1, wherein the compound or composition is for administration in combination with an anti-rejection agent selected from the group consisting of corticosteroids, calcineurin inhibitors, antiproliferative agents, monoclonal antibodies and polyclonal antibodies.

11. The compound or composition for use according to claim 10, wherein the anti-rejection agent is an antiproliferative agent.

12. The compound or composition for use according to claim 11, wherein the antiproliferative agent is sirolimus.

13. The compound or composition for use according to claim 10, wherein the anti-rejection agent is a corticosteroid.

14. The compound or composition for use according to claim 10, wherein the anti-rejection agent is a calcineurin inhibitor.

15. The compound or composition for use according to claim 14, wherein the calcineurin inhibitor is tacrolimus.

16. The compound or composition for use according to claim 10, wherein the anti-rejection agent is a monoclonal antibody or a polyclonal antibody.

17. The compound or composition for use according to any one of claims 10-16 wherein the anti-rejection agent is formulated separate from the compound or composition.

18. The compound or composition for use according to any one of claims 10-17, wherein the compound or composition is for administration sequentially with the anti-rejection agent.

19. The compound or composition for use according to claim 1, wherein the GvHD is alloantibody driven GvHD.

## Patentansprüche

1. Verbindung oder Zusammensetzung, umfassend die Verbindung, zur Verwendung bei einem Verfahren zur Behandlung oder Prävention von GvHD bei einem Säuger, wobei die Verbindung die folgende Struktur aufweist: 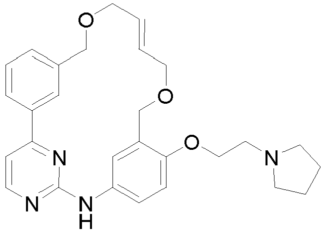 oder ein pharmazeutisch annehmbares Salz oder N-Oxid davon.

2. Verbindung oder Zusammensetzung zur Verwendung gemäß Anspruch 1, **dadurch gekennzeichnet, dass** die Verbindung oder Zusammensetzung zur gleichzeitigen Verabreichung mit einer allogenen Knochenmark- oder hämatopoetischen Stammzellentransplantation bestimmt ist.

3. Verbindung oder Zusammensetzung zur Verwendung gemäß Anspruch 1, **dadurch gekennzeichnet, dass** die Verbindung oder Zusammensetzung zur sequentiellen Verabreichung mit einer allogenen Knochenmark- oder hämatopoetischen Stammzellentransplantation bestimmt ist.

4. Verbindung oder Zusammensetzung zur Verwendung gemäß Anspruch 1, **dadurch gekennzeichnet, dass** die Verbindung oder Zusammensetzung zur Verabreichung im Anschluss an eine allogene Knochenmark- oder hämatopoetische Stammzellentransplantation bestimmt ist.

5. Verbindung oder Zusammensetzung zur Verwendung gemäß Anspruch 1, wobei die Verbindung oder Zusammensetzung zur einmal täglichen Verabreichung von Tag 1 bis etwa Tag 2000 nach einer allogenen Knochenmark- oder hämatopoetischen Stammzellentransplantation bestimmt ist.

6. Verbindung oder Zusammensetzung zur Verwendung gemäß Anspruch 1, wobei die Verbindung oder Zusammensetzung zur Verabreichung vom Beginn der GvHD-Symptome bis etwa zum Tag 2000 nach einer allogenen Knochenmark- oder hämatopoetischen Stammzellentransplantation bestimmt ist.

7. Verbindung oder Zusammensetzung zur Verwendung gemäß Anspruch 1, wobei die Verbindung oder Zusammensetzung in einer Dosierung zwischen etwa 0,1 mg/kg pro Tag bis etwa 1000 mg/kg pro Tag, bevorzugt etwa 100 mg/kg pro Tag, verabreicht wird.

8. Verbindung oder Zusammensetzung zur Verwendung gemäß Anspruch 1, wobei die Verbindung oder Zusammensetzung zur oralen Verabreichung formuliert ist.

9. Verbindung oder Zusammensetzung zur Verwendung gemäß Anspruch 1, wobei die Verbindung oder Zusammensetzung zur zweimaligen Verabreichung pro Tag formuliert ist.

10. Verbindung oder Zusammensetzung zur Verwendung gemäß Anspruch 1, wobei die Verbindung oder Zusammensetzung zur Verabreichung in Kombination mit einem Wirkstoff gegen Abstoßung, ausgewählt aus der Gruppe bestehend aus Kortikosteroiden, Calcineurininhibitoren, antiproliferativen Wirkstoffen, monoklonalen Antikörpern und polyklonalen Antikörpern, bestimmt ist.

11. Verbindung oder Zusammensetzung zur Verwendung gemäß Anspruch 10, wobei der Wirkstoff gegen Abstoßung ein antiproliferativer Wirkstoff ist.

12. Verbindung oder Zusammensetzung zur Verwendung gemäß Anspruch 11, wobei der antiproliferative Wirkstoff Sirolimus ist.

13. Verbindung oder Zusammensetzung zur Verwendung gemäß Anspruch 10, wobei der Wirkstoff gegen die Abstoßung ein Kortikosteroid ist.

14. Verbindung oder Zusammensetzung zur Verwendung gemäß Anspruch 10, wobei der Wirkstoff gegen Abstoßung ein Calcineurininhibitor ist.

15. Verbindung oder Zusammensetzung zur Verwendung gemäß Anspruch 14, wobei der Calcineurininhibitor Tacrolimus ist.

16. Verbindung oder Zusammensetzung zur Verwendung gemäß Anspruch 10, wobei der Wirkstoff gegen Abstoßung ein monoklonaler Antikörper oder ein polyklonaler Antikörper ist.

17. Verbindung oder Zusammensetzung zur Verwendung gemäß einem der Ansprüche 10-16, wobei der Wirkstoff gegen Abstoßung getrennt von der Verbindung oder Zusammensetzung formuliert ist.

18. Verbindung oder Zusammensetzung zur Verwendung gemäß einem der Ansprüche 10-17, wobei die Verbindung oder Zusammensetzung zur sequentiellen Verabreichung mit dem Wirkstoff gegen Abstoßung bestimmt ist.

19. Verbindung oder Zusammensetzung zur Verwendung gemäß Anspruch 1, wobei die GvHD eine alloantikörpergesteuerte GvHD ist.

## Revendications

1. Composé ou composition comprenant le composé pour utilisation dans un procédé de traitement ou de prévention de la maladie du greffon contre l'hôte (GVH) chez un mammifère, le composé présentant la structure suivante : 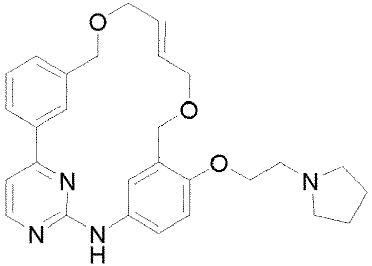 ou un sel ou N-oxyde pharmaceutiquement acceptable de celui-ci.

2. Composé ou composition pour utilisation selon la revendication 1, caractérisé(e) en ce que le composé ou la composition est destiné(e) à être administré(e) simultanément avec une greffe allogénique de moelle osseuse ou de cellules souches hématopoïétiques.

3. Composé ou composition pour utilisation selon la revendication 1, caractérisé(e) en ce que le composé ou la composition est destiné(e) à être administré(e) de manière séquentielle avec une greffe allogénique de moelle osseuse ou de cellules souches hématopoïétiques.

4. Composé ou composition pour utilisation selon la revendication 1, caractérisé(e) en ce que le composé ou la composition est destiné(e) à être administré(e) suite à une greffe allogénique de moelle osseuse ou de cellules souches hématopoïétiques.

5. Composé ou composition pour utilisation selon la revendication 1, dans lequel/laquelle le composé ou la composition est destiné(e) à être administré(e) une fois par jour du jour 1 au jour 2000 environ après une greffe allogénique de moelle osseuse ou de cellules souches hématopoïétiques.

6. Composé ou composition pour utilisation selon la revendication 1, dans lequel/laquelle le composé ou la composition est destiné(e) à être administré(e) dès l'apparition des symptômes de GVH jusqu'à environ 2000 jours après une greffe allogénique de moelle osseuse ou de cellules souches hématopoïétiques.

7. Composé ou composition pour utilisation selon la revendication 1, dans lequel/laquelle le composé ou la composition est administré(e) à une posologie comprise d'environ 0,1 mg/kg par jour à environ 1000 mg/kg par jour, de préférence d'environ 100 mg/kg par jour.

8. Composé ou composition pour utilisation selon la revendication 1, dans lequel/laquelle le composé ou la composition est formulé(e) pour être administré(e) par voie orale.

9. Composé ou composition pour utilisation selon la revendication 1, dans lequel/laquelle le composé ou la composition est formulé(e) pour être administré(e) deux fois par jour.

10. Composé ou composition pour utilisation selon la revendication 1, dans lequel/laquelle le composé ou la composition est destiné(e) à être administré(e) en combinaison avec un agent antirejet sélectionné dans le groupe consistant en des corticostéroïdes, des inhibiteurs de la calcineurine, des agents antiprolifératifs, des anticorps monoclonaux et des anticorps polyclonaux.

11. Composé ou composition pour utilisation selon la revendication 10, dans lequel/laquelle l'agent antirejet est un agent antiprolifératif.

12. Composé ou composition pour utilisation selon la revendication 11, dans lequel/laquelle l'agent antiprolifératif est le sirolimus.

13. Composé ou composition pour utilisation selon la revendication 10, dans lequel/laquelle l'agent antirejet est un corticostéroïde.

14. Composé ou composition pour utilisation selon la revendication 10, dans lequel/laquelle l'agent antirejet est un inhibiteur de la calcineurine,

15. Composé ou composition pour utilisation selon la revendication 14, dans lequel/laquelle l'inhibiteur de la calcineurine est le tacrolimus.

16. Composé ou composition pour utilisation selon la revendication 10, dans lequel/laquelle l'agent antirejet est un anticorps monoclonal ou un anticorps polyclonal.

17. Composé ou composition selon l'une quelconque des revendications 10-16, dans lequel/laquelle l'agent antirejet est formulé séparément du composé ou de la composition.

18. Composé ou composition pour utilisation selon l'une quelconque des revendications 10-17, dans lequel/laquelle le composé ou la composition est destiné(e) à être administré(e) en séquence avec l'agent antirejet.

19. Composé ou composition pour utilisation selon la revendication 1, dans lequel/laquelle la maladie du greffon contre l'hôte (GVH) est une GVH entraînée par alloanticorps.
